(19) Europäisches Patentamt
European Patent Office
Office européen des brevets

(11) Publication number: **0 189 877**
**A2**

(12) # EUROPEAN PATENT APPLICATION

(21) Application number: 86101017.1

(22) Date of filing: 25.01.86

(51) Int. Cl.⁴: C 07 D 403/14
C 07 D 241/08, C 08 K 5/00
C 08 G 73/00

(30) Priority: 29.01.85 US 696133
29.01.85 US 696123

(43) Date of publication of application: 06.08.86 Bulletin 86/32

(84) Designated Contracting States: CH DE FR GB IT LI NL

(71) Applicant: The B.F. GOODRICH Company
Dept. 0015 WHB-6 500 South Main Street
Akron, Ohio 44318(US)

(72) Inventor: Son, Pyong Nae
2106 Ayers Avenue
Akron Ohio 44313(US)

(72) Inventor: Ta-Yuan Lai, John
3465 Ridge Park Blvd.
Broadview Heights Ohio 44147(US)

(74) Representative: von Kreisler, Alek, Dipl.-Chem. et al,
Deichmannhaus am Hauptbahnhof
D-5000 Köln 1(DE)

(54) **Hindered 2-keto-1,4-diazacycloalkane oligomers containing triazine rings or ester groups and compositions stabilized thereby.**

(57) Novel hindered 2-keto-1,4-diazacycloalkane oligomers having the general formula

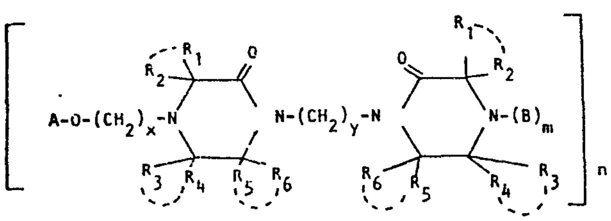

are useful antioxidants and UV stabilizers for various polymers.

EP 0 189 877 A2

0189877

# HINDERED 2-KETO-1,4-DIAZACYCLOALKANE OLIGOMERS CONTAINING TRIAZINE RINGS OR ESTER GROUPS AND COMPOSITIONS STABILIZED THEREBY

## BACKGROUND OF THE INVENTION

Organic materials, both natural and synthetic, are conventionally protected against degradation by ultraviolet (UV) radiation by incorporating a UV light stabilizer in the material. Many classes of compounds are known to be useful UV light stabilizers, with some compounds being more effective than others. Particularly effective 2-keto-diazacycloalkanes, which provide stabilized compositions that are resistant to degradation by UV light, include the 2-keto-1,4-diazacycloalkanes disclosed in U.S. Patent No. 4,190,571; and the 2-keto-1,5-diazacycloalkanes disclosed in U.S. Patent No. 4,207,228. Other 2-keto-diazacycloalkanes useful as UV light stabilizers are disclosed in U.S. Patent Nos. 3,919,234; 3,920,659, and 3,928,330 which teach substituted piperazinediones. U.S. Patent No. 4,185,003 describes hindered piperidene-esters which are used as stabilizers in thermoplatic copolyetherester elastomers.

The compounds of this invention belong to a well-recognized chemical class of UV stabilizers and antioxidants for a variety of polymers. Compounds of this class include piperidinyl-triazine derivatives such as those disclosed in U.S. Patent Nos. 4,386,177; 4,356,287; 4,321,374; 4,294,963; 4,234,728 and 4,108,829. The above patents describe oligomers of triazine rings with monoaza-ring substituents, and teach the particular use of these oligomers as light stabilizers in polyolefins. The 2-keto-1,4-diazacycloalkane oligomers of the present

invention contain 2-keto-diaza rings in the oligomeric backbone which are bonded to the triazine rings through an ether linkage. The compounds of this invention further include compounds of bis(2-keto-1,4-diazacycloalkanes) containing ester groups in the structural backbone. The novel compounds of this invention are excellent stabilizers for polyolefins and exhibit resistance to solvent extraction.

It is an object of the present invention to provide novel 2-keto-1,4-diazacycloalkane oligomers containing triazine rings or ester groups which are useful as stabilizers for a variety of organic materials. It is a further object of this invention to provide novel 2-keto-diazacycloalkane oligomers containing triazine rings or ester groups which are useful as UV stabilizers and as antioxidants for various polymers. It is an even further object of this invention to provide novel 2-keto-diazacycloalkane oligomers containing triazine rings or ester groups and stabilized compositions which are resistant to solvent extraction.

SUMMARY OF THE INVENTION

Novel hindered 2-keto-1,4-diazacycloalkane oligomers containing triazine rings or ester groups are useful antioxidants and UV stabilizers for organic materials such as polyolefins. The novel keto-diazacycloalkane oligomer stabilizer compounds of this invention may be represented by the general formula

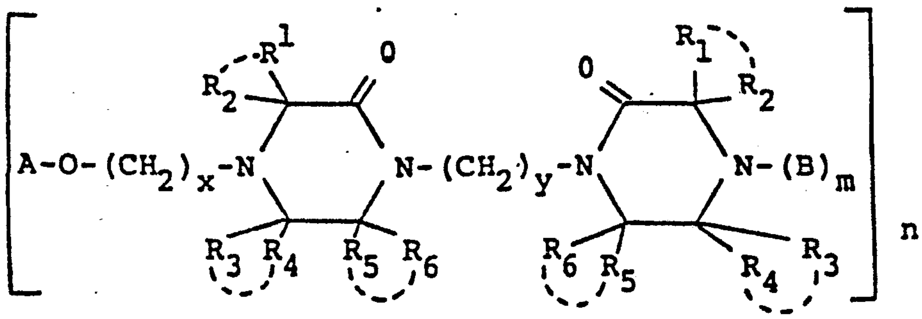

wherein B represents

$$-(CH_2)_2\,O\overset{O}{\overset{\|}{C}}R' \quad \text{or} \quad -(CH_2)_z- \;;$$

x, y and z independently is an integer from 1 to about 10, m is 0 or 1, and R' is alkyl, aralkyl or hydroxyaralkyl; n ranges from 1 to about 20; $R_1$, $R_2$, $R_3$ and $R_4$ each independently represent alkyl or haloalkyl having from 1 to about 12 carbon atoms, cyanoalkyl, aminoalkyl or iminoalkyl having from 2 to about 12 carbon atoms, cycloalkyl or hydroxy-cycloalkyl having from 5 to about 14 carbon atoms, alkenyl or aralkyl having from 7 to about 14 carbon atoms, alkylene having from 2 to about 7 carbon atoms, and $R_1$ and $R_2$ or $R_3$ and $R_4$ in combination with each other represent cycloalkyl having from 5 to about 14 carbon atoms; $R_5$ and $R_6$ are the same as $R_1$-$R_4$ hereinabove and additionally may be hydrogen; A is

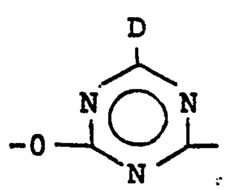

wherein D is piperidinyl, morpholinyl, alkylamino or dialkylamino having from 2 to about 12 carbon atoms.

A may be

$$\overset{\overset{O}{\|}}{C}=R'$$

wherein R' is defined as above. A may be

$$-\overset{\overset{O}{\|}}{C}-O\overset{\overset{O}{\|}}{C}-(CH_2)_p\left[\overset{\overset{O}{\|}}{C}O-J-O\overset{\overset{O}{\|}}{C}-(CH_2)_p\right]_q$$

p is 2 to about 18, q is 0 or 1, and J is arylene, aralkylene, cycloalkylene or bicyclodiyl.

## DETAILED DESCRIPTION

This invention relates to novel hindered 2-keto-1,4-diazacycloalkane oligomers containing triazine rings or ester groups and compositions stabilized thereby. The novel keto-diazacycloalkane oligomers containing triazine rings or ester groups are useful as UV stabilizers and antioxidants for a variety of organic materials. They are particularly useful as UV light stabilizers in compositions subject to UV light degradation. As stabilizers they are used in the range from about 0.01 to about 10 parts by weight, and preferably from about 0.1 to about 1.0 part, per 100 parts by weight of organic material. These organic materials may be low or high molecular weight materials, and particularly include homopolymers, copolymers and mixtures thereof. Examples of materials that can be stabilized by the oligomers of this invention include oils; monomers, such as α,β-olefinically unsaturated acrylates, dienes, vinyl nitriles and the like; alcohols; aldehydes; and natural and synthetic polymers. Examples of known polymeric materials which can be stabilized by the keto-diazacycloalkane oligomers of

this invention include natural rubber, synthetic rubbers (such as cis-polyisoprene, styrene-butadiene rubber, dienenitrile rubbers), polyepihalohydrin polymers, polyurethanes, polyvinyl chloride resins, acrylonitrile-butadiene-styrene resins, polystyrene, polyacrylonitrile, polymethacryates, polycarbonates, varnish, phenol-formaldehyde resins, polyepoxides, polyesters, and polyolefin homo and copolymers such as polyethylene, polypropylene, ethylene-propylene polymers, ethylene-propylenediene polymers, ethyl-vinyl acetate polymers, and the like.

The novel 2-keto-1,4-diazacycloalkane oligomers of the present invention have the structural formula:

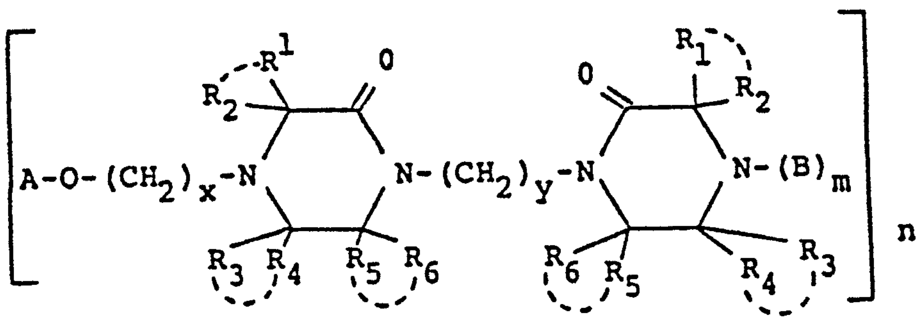

wherein B represents

$$-(CH_2)_2OCR' \text{ (with } \overset{O}{\parallel} \text{ on C) or } -(CH_2)_z-;$$

x, y, and z independently is an integer from 1 to about 10; m is 0 or 1, and R' is alkyl, aralkyl or hydroxyaralkyl; n ranges from 1 to about 20; $R_1$, $R_2$, $R_3$ and $R_4$ each independently represent alkyl or haloalkyl having from 1 to about 12 carbon atoms, cyanoalkyl, aminoalkyl or iminoalkyl having from 2 to about 12 carbon atoms, cycloalkyl or hydroxy-cycloalkyl having from 5 to about 14 carbon atoms, alkenyl or aralkyl having from 7 to about 14

carbon atoms, alkylene having from 2 to about 7 carbon atoms, and $R_1$ and $R_2$ or $R_3$ and $R_4$ in combination with each other represent cycloalkyl having from 5 to about 14 carbon atoms; $R_5$ and $R_6$ are the same as $R_1$-$R_4$ hereinabove and additionally may be hydrogen; A is

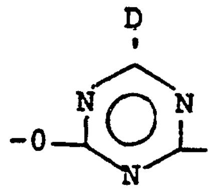

wherein D is piperidinyl, morpholinyl, dialkylamino or alkylamino having from 2 to about 12 carbon atoms. A may be

$$\overset{O}{\overset{\|}{C}}-R'$$

wherein R' is defined above. A may be

$$-\overset{O}{\overset{\|}{C}}-O\overset{O}{\overset{\|}{C}}-(CH_2)_p+\overset{O}{\overset{\|}{C}}O-J-O\overset{O}{\overset{\|}{C}}-(CH_2)_p+_q$$

p is 2 to about 18, q is O or 1 and J is arylene, aralkylene, cycloalkylene, or bicyclodial. Preferably, $R_1$, $R_2$, $R_3$, and $R_4$ are methyl and $R_5$ and $R_6$ are hydrogen and R' contains from 1 to about 20 carbons. J preferably contains from 2 to about 40 carbon atoms.

Preferably, the novel 2-keto-1,4-diazocycloalkane oligomers containing ester groups of the present invention have the

structural formula

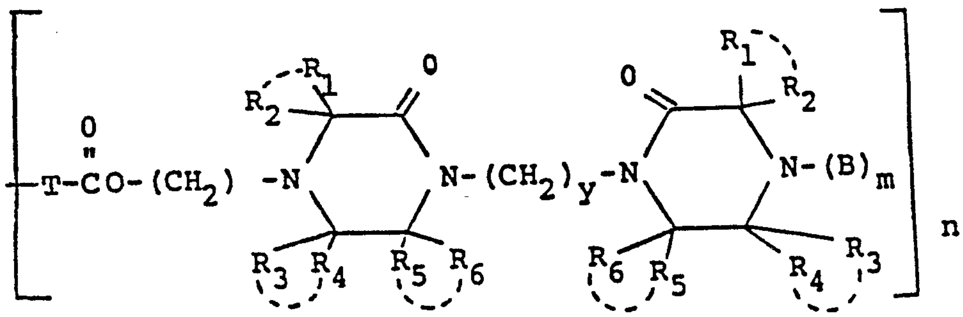

wherein B represents $-(CH_2)_2O\overset{O}{\overset{\|}{C}}R'$ or $-(CH_2)_2-$, m is 0 or 1, and R' is alkyl, aralkyl or hydroxyaralkyl; T represents R' as defined above or

$$-O\overset{O}{\overset{\|}{C}}-(CH_2)_p\left[\overset{O}{\overset{\|}{C}}O-J-O\overset{O}{\overset{\|}{C}}-(CH_2)_x\right]_q ,$$

Preferably, the novel 2-keto-1,4-diazacycloalkane oligomers containing triazine rings of the present invention have the structural formula

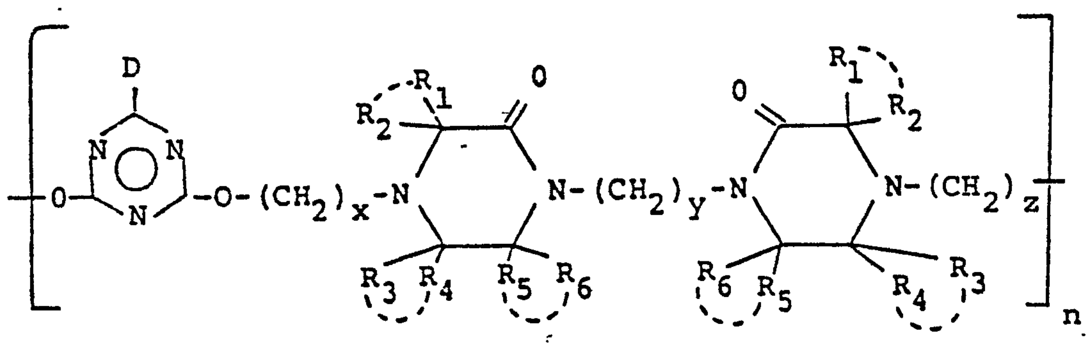

Compounds falling within the scope of this invention include, for example, poly[oxy[6-(1,1,3,3-tetramethylbutyl)-

amino]-1,3,5-triazine-2,4-diyloxy-1,2-ethanediyl (2,2,6,6-tetramethyl-3-oxo-1,4-piperazinediyl)-1,2-ethanediyl(3,3,5,5-tetramethyl-2-oxo-1,4-piperazine-diyl)-1,2-ethanediyl];

poly[oxy[6-(1-piperidinyl]-1,3,5-triazine-2,4-diyloxy-1,2-ethanediyl(2,2,6,6-tetramethyl-3-oxo-1,4-piperazinediyl)-1,2-ethanediyl(3,3,5,5-tetramethyl-2-oxo-1,4-piperazinediyl)-1,2-ethanediyl];

1,2-ethanediylbis[(2,2,6,6-tetramethyl-3-oxo-4,1-pipera zinediyl)-2,1-ethanediyl] ester of 3,5-bis(1,1-dimethylethyl)-4-hydroxybenzene-propionic acid;

1,2-ethanediylbis[(2,2,6,6-tetramethyl-3-oxo-4,1-piperi dinyl)-2,1-ethanediyl] ester of stearic acid;

poly[oxycarbonyl-1,2-ethanediylcarbonyloxy-1,2-ethanedi yl(2,2,6,6-tetramethyl-3-oxo-1,4-piperazinediny)-1,2-et hanediyl(3,3,5,5-tetramethyl-2-oxo-1,4-piperazinediyl)-1,2-ethanediyl];

poly[oxycarbonyl-1,8-octanediylcarbonyloxy-1,2-ethanedi yl(2,2,6,6-tetramethyl-3-oxo-1,4-piperazinediyl)-1,2-et hanediyl(3,3,5,5-tetramethyl-2-oxo-1,4-piperazinediyl)-1,2-ethanediyl];

poly[oxycarbonyl-1,3-propanediylcarbonyloxy-1,2-ethaned iyl(2,2,6,6-tetramethyl-3-oxo-1,4-piperazinediyl)-1,2-e thanediyl(3,3,5,5-tetramethyl-2-oxo-1,4-piperazinediyl) -1,2-ethanediyl]; and the like.

The novel compounds of this invention can be prepared by reaction of substituted 2-keto-1,4-diazacycloalkanes with substituted trazines in the presence of alkali metal or with the appropriate ester in the presence of a catalyst. Suitable substituted 2-keto-1,4-di-azacycloalkanes for use in this invention include those disclosed in U.S. Patent No. 4,167,512, which is herein incorporated by reference. Suitable substituted triazines include

the 1,3,5-triazines which are known in the art and particularly described in various U.S. patents, such as U.S. Patent Nos. 4,356,287; 4,108,829; and 4,234,728, which are herein incorporated by reference. The reaction may be carried out in the presence of inert solvents such as chlorobenzene, acetone, dioxane, toluene, xylene and the like. Suitable esters include alkyl, hydroxyalkyl aryl, aralkyl, hydroxyaralkyl, cycloalkylene, and bicyclidiyl esters. Suitable esters include, for example, methyl stearate, dimethyl succinate, dimethyl sebacate, dimethyl glutarate, 3,5-bis(1,1-dimethylethyl)-4-hydroxybenzenepropanoate, and the like. Suitable catalysts include, for example, organo-metallic compounds such as tetra-isopropyl-orthotitanate and potassium tert-butoxide. The reaction may be carried out in the presence of inert solvens such as dichlorobenzene, chlorobenzene, hexane, toluene, xylene, acetone, dioxane, and the like.

The novel keto-diazacycloalkane oligomers of this invention may be used to stabilize organic materials either alone or in combination with other secondary stabilizers. Particularly desirable secondary stabilizers are one or more antioxidants used in the range from about 0.1 part to about 20 parts by weight, preferably from about 0.1 part to about 5 parts by weight per 100 parts by weight of the material. Of the types of antioxidants to be used, phosphite, phosphate, sulfide and phenolic antioxidants are preferred. Most preferred are phenolic antioxidants such as
2,6-di-t-butyl-paracresol;
2,2'-methylene-bis-(6-t-butyl-phenol);
2,2'-thiobis-(4-methyl-6-t-butyl-phenol);

2,2'-methylene-bis-(6-t-butyl-4-ethyl-phenol); 4,4'-butylene-bis(6-t-butyl-m-cresol); 2-(4-hydroxy-3,5-di-t-butylanilino)-4,6-bis-(octylthio) -1,3,5-triazine; tris 2-[ß-(3,5-di-tert-butyl-4-hydroxyphenyl)propionoxy]ethyl isocyanurate; tris-(3,5-di-t-butyl-4-hydroxybenzyl)isocyanurate; tetrakis[methylene(3,5-di-tert-butyl-4-hydroxyhydrocinnamate)]methane ;tris 2-[ß-(3,5-di-tert-butyl-4-hydroxyphenyl)propionoxy]ethyl isocyanurate; 1,3,5-tris-(3,5-di-t-butyl-4-hydroxybenzyl)isocyanurate ; 2,2',2"-tris[3-(3,5-di-t-butyl-4-hydroxyphenyl) propionyloxy]ethylisocyanurate; and other antioxidant synergists such as distearyl thiodipropionate; dilauryl thiodipropionate; tri(nonylphenyl)phosphite; and tin thioglycolate. Other ingredients such as lubricants, plasticizers, pigments, tackifiers, fillers, flame retardants, fungicides, and the like may also be added.

The substituted keto-diazacycloalkane oligomer stabilizers, and other compounding ingredients, if used, can be admixed with organic materials using known mixing techniques and equipment such as internal mixing kettles, a Banbury mixer, a Henschel mixer, a two-roll mill, an extruder mixer, or other standard equipment, to yield a composition which may be extruded, pressed, blowmolded or the like into film, fiber or shaped articles. Usual mixing times and temperatures can be employed which may be determined with a little trial and error for any particular composition. The objective is to obtain intimate and uniform mixing of the components.

To further illustrate the present invention, the following specific examples are given, it being

understood that this is merely intended in an illustrative and not a limitative sense.

EXAMPLE I

Preparation of poly[oxy[6-[1,1,3,3-tetramethylbutyl)amino]-1,3,5-triazine-2,4-diyloxy-1,2-ethanediyl(2,2,6,6-tetramethyl-3-oxo-1,4-piperazine-diyl)-1,2-ethanediyl(3,3,5,5-tetramethyl-2-oxo-1,4-piperazine-diyl)-1,2-ethanediyl].

Into a 500 ml three-necked flask was placed 200 ml of dry xylenes and 4 drops of oleic acid. The above mixture was heated under a nitrogen blanket. When the temperature of the mixture reached 50°C, 0.92 grams of sodium metal was added. When the temperature of the mixture reached 105°C, 8.53 grams of 1,1'-(1,2-ethanediyl)bis[4-(2-hydroxyethyl)-3,3,5,5-tetramethylpiperazinone] was added over a 12 minute period. After refluxing overnight, 5.54 grams of 2,4-dichloro-6-(1,1,3,3-tetramethylbutyl)amino-1,3,5-triazine was added, and the mixture was refluxed for approximately 5 hours. About 50 ml of ethanol was added to the mixture and it was allowed to stir overnight at room temperature. The mixture was stripped to obtain a straw-colored solid, which was washed with 300 ml of water and isolated by filtration. After drying, 12.5 grams of solid product was obtained having a softening point range of 103-116°C and a number average molecular weight (Mn) of 1128. The product was identified as having the following structure

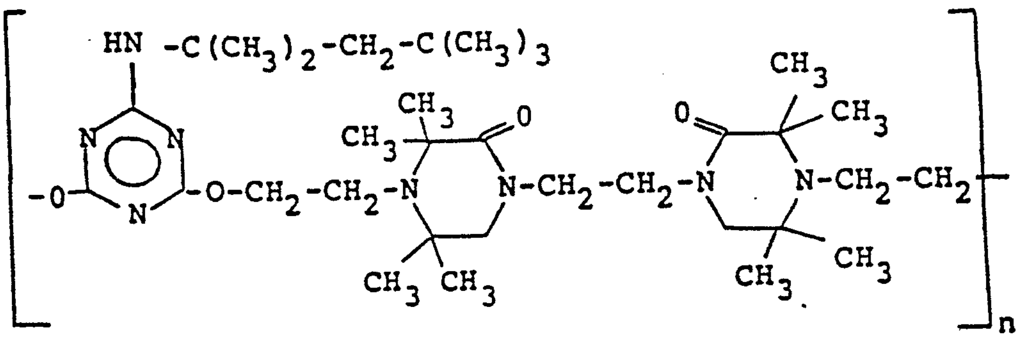

EXAMPLE II

Preparation of poly[oxy[6-(1-piperidinyl)-1,3,5-triazine-2,4-diyl]oxy-1,2-ethanediyl(2,2,6,6-tetramethyl-3-oxo-1,4-piperazinediyl)-1,2-ethanediyl (3,3,5,5-tetramethyl-2-oxo-1,4-piperazinediyl)-1,2-ethanediyl].

Into a 2-neck 100 ml flask, equipped with magnetic stirrer, condenser,and a Dean-Stark trap, were placed 8.53 grams of 1,1'-(1,2-ethanediyl)bis[4-(2-hydroxyethyl)-3,3,5,5-tetramethylpiperazinone], 4.66 grams of 2,4-dichloro-6-(1-piperidinyl)-1,3,5-triazine, 50 ml of chlorobenzene, and 1.68 grams of sodium hydroxide in 5 ml of water. The above mixture was heated to reflux and about 5.5 ml of water was collected. After refluxing overnight the reaction was terminated and the mixture cooled. After stripping, a solid product was obtained which was washed with 150 ml of water and isolated by filtration. The product was dried to yield 11.0 grams of a white solid having a softening point of about 115°C and Mn 1341. The product was identified as having the following structure

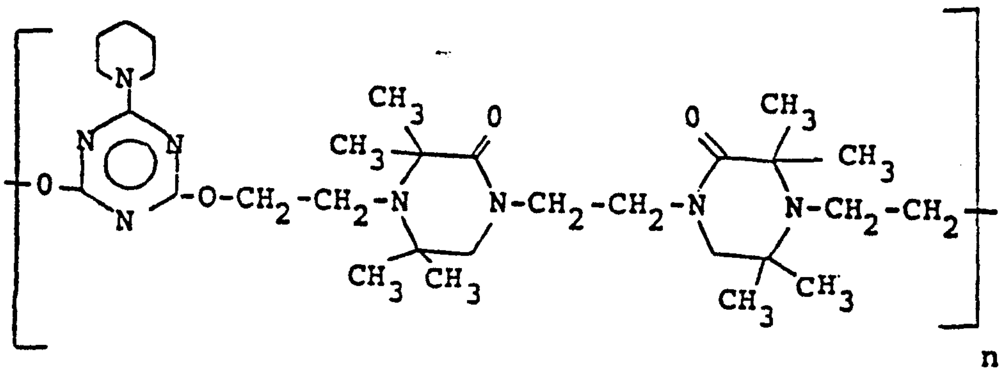

EXAMPLE III

The compounds prepared above were tested for their effectiveness as UV stabilizers in 2 mil (0.0254 mm) polypropylene strips. In each sample 0.05 parts by weight of tris 2-[ß-(3,5-di-tert-butyl-4-hydroxyphenyl)propionoxyoxy]ethyl isocyanurate per 100 parts by weight polypropylene was added as a secondary stabilizer. The samples were placed in a Xenon Weatherometer and the time period (failure time) measured for loss of 50% of original tensile strength. The results are presented in Table I below:

TABLE I

| Sample | UV Stabilizer(pbw) | Failure Time(hours) |
|---|---|---|
| Control | None | 260 |
| Example I | 0.1 | 770 |
| Example II | 0.1 | 820 |

(pbw is parts by weight)

The above results demonstrate the effectiveness of the compounds of this invention as UV stabilizers, particularly as synergist for conventional polymer stabilizers.

0189877

EXAMPLE IV

Preparation of 1,2-ethanediylbis[(2,2,6,6-tetramethyl-3-oxo-4,1-piperazinediyl)-2,1-ethanediyl] ester of 3,5-bis(1,1-dimethylethyl)-4-hydroxybenzene propanoic acid.

Into a 150 ml flask with a 4-neck flask head was placed 96.9 grams of ethyl 3,5-bis(1,1-diemthylethyl)-4-hydroxybenzenepropanoate. The contents were heated to 140°C and 33.7 grams of 1,1'-(1,2-ethanediyl)bis[4-(2-hydroxyethyl)-3,3,5,5-tetramethylpiperazinone] was added. The mixture was further heated to about 183°C and 0.5 ml of tetraisopropyl orthotitanate was added. After collecting about 8 ml of ethanol, the reaction temperature was maintained at about 180°C for approximately one hour and the reaction then terminated. The resulting taffy reaction mixture was placed in 600 ml of hot hexane and stirred for 5 minutes. A tan solid product was isolated from the slurry and washed with 1 liter of water. The product was dried overnight to obtain 66.5 grams of crude product.

An analytical sample of the product was prepared by recrystallizing the crude product from 1-pentanol, followed by 2 additional recrystallizations from toluene. The product had a melting point range from 174 to 176.5°C and had the following structure

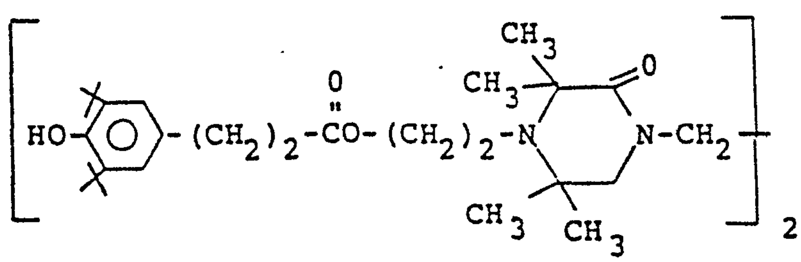

EXAMPLE V

Preparation of 1,2-ethanediylbis[2,2,6,6-tetramethyl-3-oxo-4,1-piperidinyl)-2,1-ethanediyl] ester of stearic acid.

Following the same general procedures as desacribed in Example I above, 94.4 grams of methyl stearate was reacted with 33.7 grams of 1,1'-(1,2-ethanediyl)bis[4-(2-hydroxyethyl)-3,3,5,5-tetramethylpiperazinone] in the presence of 0.5 ml of tetraisopropyl orthotitanate. The crude product was purified by recrystallizing from ethyl acetate and then from hexane-toluene. The product had a melting point of 73 - 76°C and had the following structure

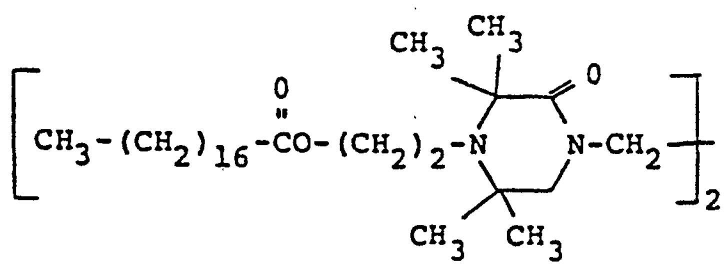

EXAMPLE VI

Preparation of poly[oxycarbonyl-1,2-ethanediylcarbonyloxy-1,2-ethanediyl(2,2,6,6-tetramethyl-3-oxo-1,4-piperazinediyl)-1,2-ethanediyl(3,3,5,5-tetramethyl-2-oxo-1,4-piperazinediyl)1,2-ethanediyl].

Into a 100 ml 3-necked flask were placed 10.66 grams of 1,1'-(1,2-ethanediyl)bis[4-(2-hydroxyethyl)-3,3,5,5-tetramethylpiperazinone], 4.02 grams of dimethyl succinate, 50 ml of dichlorobenzene, and 1.0 gram of potassium tert-butoxide. The above mixture was heated and maintained at 130 - 140°C for about 4

hours, and then reacted overnight at about 120°C. The reaction terminated the following day. The resulting thick syrup was titrated in water and then dissolved in hot toluene. After stripping the toluene, a light yellow taffy remained which was treated with hot hexane. The hexane insoluble product was isolated and analyzed. The product had a number average molecular weight (Mn) of 1870 and had the following structure

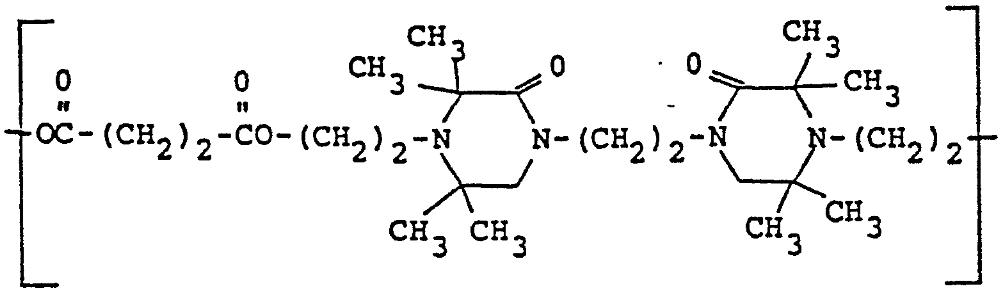

EXAMPLE VII

Preparation of poly[oxycarbonyl-1,8-octanediylcarbonyloxy-1,2-ethanediyl(2,2,6,6-tetramethyl-3-oxo-1,4-piperazinediyl)-1,2-thanediyl(3,3,5,5-tetramethyl-2-oxo-1,4-piperazinediyl)-1,2-ethanediyl].

Following the same general procedures as described in Example III above, 14.3 grams of 1,1'-(1,2-ethanediyl)-bis[4-(2-hydroxyethyl)-3,3,5,5-tetramethylpiperazinone] was reacted with 7.8 grams of dimethyl sebacate in the preence of 65 ml of dichlorobenzene and 1.5 grams of potassium tertbutoxide. The resulting syrup was dissolved in 200 ml of methylene chloride and washed with 200 ml of water. The organic layer was separated, dried over anhydrous sodium sulfate, and stripped on a

rotary evaporator to isolate 12.4 grams of the final product. The product had Mn of 1430 and the following structure

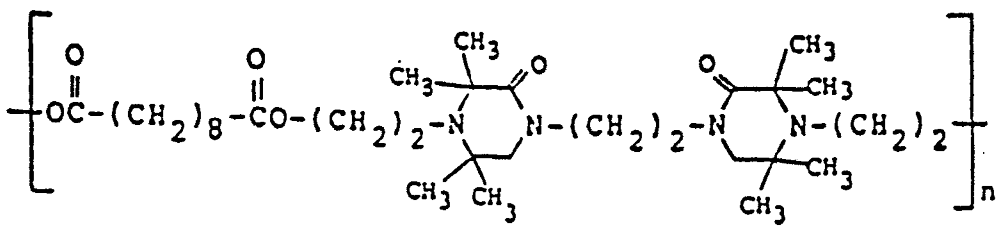

EXAMPLE VIII

Preparation of poly[oxycarbonyl-1,3-propanediylcarbonyloxy-1,2-ethanediyl(2,2,6,6-tetramethyl-3-oxo-1,4-piperazinediyl)-1,2-ethanediyl(3,3,5,5-tetramethyl-2-oxo-1,4-piperazinediyl)-1,2-ethanediyl].

Following the same general procedures as described in Example III abaove, 14.9 grams of 1,1'-(1,2-ethanediyl)bis[4-(2-hydroxyethyl)-3,3,5,5-tetramethylpiperazinone] was reacted 6.3 grams of dimethyl glutarate in the presence of 65 ml of dichlorobenzene and 1.5 grams of potassium tertbutoxide. The resulting reaction product was dissolved in 200 ml of methylene chloride and washed with 200 ml of water. The organic layer was separated, dried over sodium sulfate, and stripped to isolate 13.9 grams of resinous product. The final product had a Mn of 1260 and had the following structure

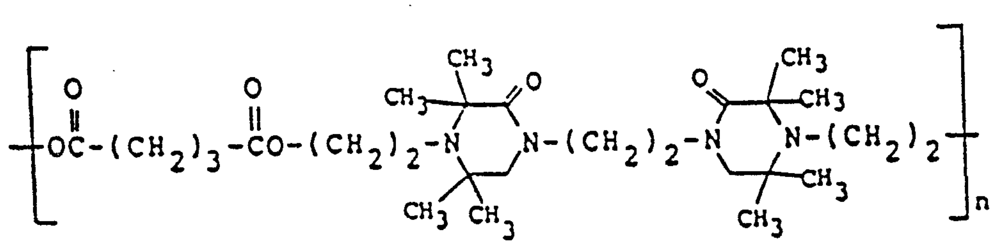

EXAMPLE IX

The compounds prepared above were tested for their effectiveness as UV stabilizers and antioxidants in 2 mil (0.0254 mm) polypropylene strips. In each sample, except Sample #3, 0.05 parts by weight of 2, 2', 2"-tris[3,5-di-t-butyl-4-hydroxyphenyl)propionyloxy]ethylisocyanurate per 100 parts by weight polypropylene was added as a secondary stabilizer. The samples were placed in a Xenon Weatherometer and an oven and the time period (failure time) measured for loss of 50% of original tensile strength. The results are presented in Table II below.

TABLE II

| Sample | UV Stabilizer (pbw)* | Xenon Weather-ometer Failure Time (hrs.) | Oven Aging (125°C) Failure Time (days) |
|---|---|---|---|
| 1. Control | none | 260 | 16 |
| 2. Example IV | 0.1 | 720 | 16 |
| 3. Example IV | 0.1 | 1480 | 16 |
| 4. Example V | 0.1 | 920 | 37 |
| 5. Example VII | 0.1 | 740 | 19 |
| 6. Example VII | 0.1 | 1000 | 31 |
| 7. Example VIII | 0.1 | 970 | 31 |

*pbw is parts by weight

The above results demonstrate the effectiveness of the compounds of this invention as antioxidants and UV stabilizers.

CLAIMS

1. A compound of the formula

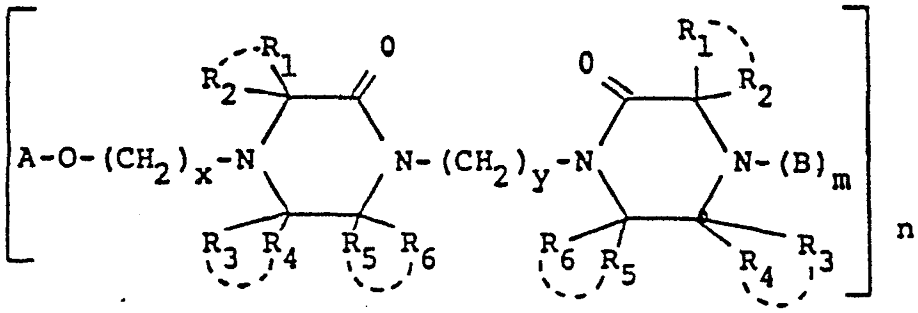

where B represents $-(CH_2)_2O\overset{O}{\overset{\|}{C}}R'$ or $-(CH_2)_z$; x, y, and z independently is an integer from 1 to about 10; m is 0 or 1, and R' is alkyl, aralkyl or hydroxyaralkyl; n ranges from 2 to about 20; $R_1$, $R_2$, $R_3$ and $R_4$ each independently represent alkyl or haloalkyl having from 1 to about 12 carbon atoms, cyanoalkyl, aminoalkyl or iminoalkyl having from 2 to about 12 carbon atoms, cycloalkyl or hydroxy-cycloalkyl having from 5 to about 14 carbon atoms, alkenyl or aralkyl having from 7 to about 14 carbon atoms, alkylene having from 2 to about 7 carbon atoms, and $R_1$ and $R_2$ or $R_3$ and $R_4$ in combination with each other represent cycloalkyl having from 5 to about 14 carbon atoms; $R_5$ and $R_6$ are the same as $R_1$-$R_4$ hereinabove and additionally may be hydrogen; A is

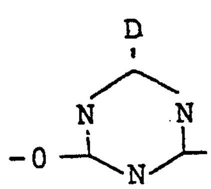

wherein D is piperidinyl, morpholinyl, alkylamino or dialkylamino having from 2 to about 12 carbon atoms; or A is

$$\overset{\overset{O}{\|}}{C}-R'$$

wherein R' is defined as above; or A is

$$\overset{\overset{O}{\|}}{C}-O\overset{\overset{O}{\|}}{C}-(CH_2)_p\!+\!\overset{\overset{O}{\|}}{C}O-J-O\overset{\overset{O}{\|}}{C}-(CH_2)_p\!+_q$$

wherein p is 2 to about 18, q is 0 or 1, and J is arylene, aralkylene, cycloalkylene or bicyclodiyl; or A is R' as defined above.

2. A compound of Claim 1 wherein B is $-(CH_2)_z$ and A is

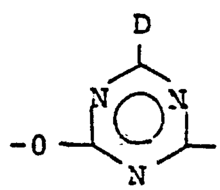

3. A compound of Claim 2 wherein $R_1$, $R_2$, $R_3$ and $R_4$ are methyl, $R_5$ and $R_6$ are hydrogen.

4. A compound of Claim 1 wherein B represents

$$-(CH_2)_2O\overset{\overset{O}{\|}}{C}R'$$

or $(CH_2)_2$ and A represents R' as defined above or A represents

$$\overset{\overset{O}{\|}}{C}-O\overset{\overset{O}{\|}}{C}-(CH_2)_p\!+\!\overset{\overset{O}{\|}}{C}O-J-O\overset{\overset{O}{\|}}{C}-(CH_2)_p\!+_z$$

5. A compound of Claim 4 wherein $R_1$, $R_2$, $R_3$ and $R_4$ are methyl and $R_5$ and $R_6$ are hydrogen.

6. A stabilized composition of matter comprising an organic material subject to ultraviolet light degradation having dispersed therein from about 0.01 part to about 10 parts by weight, per 100 parts by weight of said organic material, of a stabilizer compound of the formula:

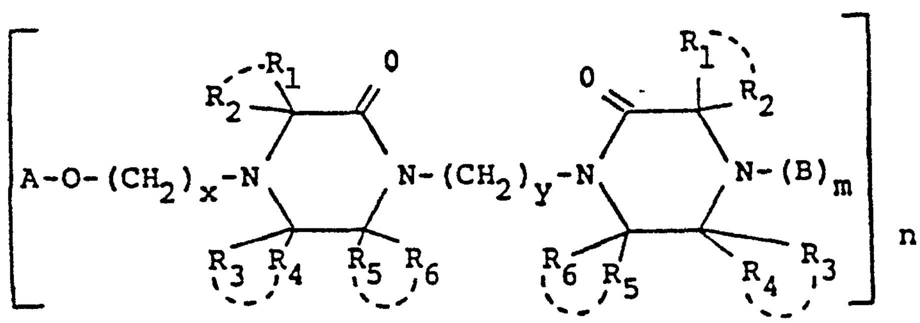

wherein B represents $-(CH_2)_2OCR'$ or $-(CH_2)_z$; wherein x, y, and z independently is an integer from 1 to about 10; m is 0 or 1, and R' is alkyl, aralkyl or hydroxyaralkyl; n ranges from 2 to about 20; $R_1$, $R_2$, $R_3$ and $R_4$ each independently represent alkyl or haloalkyl having from 1 to about 12 carbon atoms, cyanoalkyl, aminoalkyl or iminoalkyl having from 2 to about 12 carbon atoms, cycloalkyl or hydroxy-cycloalkyl having from 5 to about 14 carbon atoms, alkenyl or aralkyl having from 7 to about 14 carbon atoms, alkylene having from 2 to about 7 carbon atoms, and $R_1$ and $R_2$ or $R_3$ and $R_4$ in combination with each other represent cycloalkyl having from 5 to about 14 carbon atoms; $R_5$ and $R_6$ are the same as $R_1$-$R_4$ hereinabove and additionally may be hydrogen; and A is

0189877

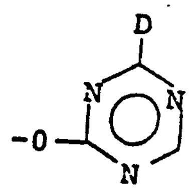

wherein D is piperidinyl, morpholinyl, dialkylamino or alkylamino having from 2 to about 12 carbon atoms.

7. A composition of Claim 6 wherein B is $-(CH_2)_z$ and A is

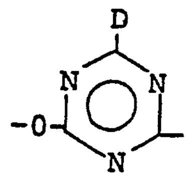

8. A composition of Claim 7 wherein $R_1$, $R_2$, $R_3$ and $R_4$ are methyl, $R_5$ and $R_6$ are hydrogen.

9. A composition of Claim 6 wherein B is

$$-\!\left(CH_2\right)_2O\overset{O}{\overset{\|}{C}}R' \quad \text{or} \quad (CH_2)_2$$

and A is R' as defined above or

$$\overset{O}{\overset{\|}{C}}-O\overset{O}{\overset{\|}{C}}-(CH_2)_p \quad -\!\!\left[\overset{O}{\overset{\|}{C}}O-J-O\overset{O}{\overset{\|}{C}}-(CH_2)_p\right]_z$$

10. A composition of Claim 9 wherein $R_1$, $R_2$, $R_3$ and $R_4$ are methyl and $R_5$ and $R_6$ are hydrogen.